# EUROPEAN PATENT SPECIFICATION

(11) **EP 2 091 457 B1**
(45) Date of publication and mention of the grant of the patent: **22.02.2017**
(21) Application number: 07849377.2
(22) Date of filing: 07.12.2007
(51) Int. Cl.: A61B 18/20, A61B 5/107

(54) **DEVICE AND METHOD FOR IMAGING SKIN OBJECTS, AND A METHOD AND DEVICE FOR REDUCING HAIR GROWTH BY MEANS THEREOF**
VORRICHTUNG UND VERFAHREN ZUR BILDDARSTELLUNG VON HAUTOBJEKTEN UND VERFAHREN UND VORRICHTUNG ZUR REDUZIERUNG DES HAARWUCHSES DAMIT
DISPOSITIF ET PROCÉDÉ POUR FORMER DES IMAGES D'OBJETS CUTANÉS, ET PROCÉDÉ ET DISPOSITIF POUR RÉDUIRE LA CROISSANCE PILEUSE AU MOYEN DE CEUX-CI

(30) Priority: 12.12.2006 EP 06125915
(43) Date of publication of application: 26.08.2009
(73) Proprietor: Koninklijke Philips N.V., 5656 AE Eindhoven (NL)
(72) Inventor: VERHAGEN, Rieko, 5656 AE Eindhoven (NL); SPIKKER, Bart W. J., 5656 AE Eindhoven (NL); VAN HAL, Robbert A. M., 5656 AE Eindhoven (NL); KHARIN, Aleksey, 5656 AE Eindhoven (NL); UZUNBAJAKAVA, Natallia, 5656 AE Eindhoven (NL)
(74) Representative: de Haan, Poul Erik
(86) International application number: PCT/IB2007/054969
(87) International publication number: WO 2008/072151

(56) References cited:
- WO-A-2004/008114
- WO-A-2005/102153
- US-A- 5 386 317
- US-A- 5 788 639
- PELI E: "CIRCULAR POLARIZERS ENHANCE VISIBILITY OF ENDOTHELIUM IN SPECULAR REFELCTION BIOMICROSCOPY" ARCHIVES OF OPHTHALMOLOGY, XX, XX, vol. 103, 1 May 1985 (1985-05-01), pages 670-672, XP008024372 ISSN: 0003-9950

## Description

### FIELD OF THE INVENTION

The present invention relates to a device for reducing growth of hairs, comprising a hair growth reducing means that is arranged to supply an amount of optical energy to at least a portion of at least one hair that is sufficient to affect the hair's integrity, and further comprising a device for imaging hairs near a skin surface of a body part, wherein the hair growth reducing means is operatively coupled to the device for imaging hairs.

### BACKGROUND OF THE INVENTION

Document WO 2005/102153 A1 dicloses a device of the kind mentioned in the opening paragraph having a hair detection device with a source of electromagnetic radiation and an imaging sensor, and with radiation selection means. The device couples the radiation into the skin, and said radiation reaches the sensor after multiple scattering. The radiation selection means improves a ratio between said multiply scattered radiation and other, unwanted radiation. The radiation selection means may comprise complementary linear polarizers.

It was found in practice that such devices do not always provide a reliable imaging of hairs below the skin surface. In some cases, images were very weak, with a low contrast between hairs and surrounding tissue. A reliable hair detection is not always possible in particular because of the relatively weak signals that can be extracted from below the skin surface, and because automated hair detection requires a good contrast.

WO 2004/008114 A1 discloses a method for the differentiated, target structure-dependent illumination and viewing of a surface. According to this method, the surface is illuminated with elliptically polarized light, and the light reflected by the surface is blocked or allowed to pass by at least one observation system containing an analyzer, in order to view the light reflected by the surface or scattered on a substructure of said surface as a high-lighted feature. In particular, the method is used for viewing blood vessels present in the skin and for skin surface structures like skin wrinkles.

US Patent 5,788,639 discloses a confocal imaging system for in vivo observation of dermal and subdermal tissue, which allows the diagnosis of conditions substantially beneath the surface of the skin. A confocal head has optics which scan the tissue so as to provide images of vertical sections of the tissue. Both two and three dimensional imaging is provided for diagnosis and location of basal cell carcinomas and melanomas so as to enable visualization of tumor borders. The system has a quarter wave plate to convert incident linear polarization from a laser to circular polarization, and to convert a coherent component of light scattered by the skin into linear polarization. A beam splitter assembly directs the coherent light component of the scattered light towards a photodetector.

### OBJECT OF THE INVENTION

It is an object of the present invention to provide a device of the kind mentioned in the opening paragraph that renders possible a more reliable imaging of hairs.

In the present invention, a 'hair near a skin surface' is a hair that is present on the skin or (slightly) above or beneath the surface of the skin.

### SUMMARY OF THE INVENTION

The above object is achieved with a device according to the invention, characterized in that the device for imaging hairs comprises a source arranged to emit optical radiation having a wavelength between 600 and 2000 nm and a linear polarization state, a detector arranged to detect optical radiation returning from said hair, an elliptical polarization means positioned in an optical path of the optical radiation between the source and said skin surface, and a ratio increaser means that is arranged to increase a ratio of optical radiation that returns from said hairs to optical radiation that returns from said skin surface, wherein said ratio increaser means is positioned in an optical path of the optical radiation between said skin surface and said detector and comprises either said elliptical polarization means and a linear polarizer arranged in the optical path between the detector and said elliptical polarization means to suppress or reflect radiation having a polarization state orthogonal to said linear polarization state, or a second elliptical polarization means and a linear polarizer arranged in the optical path between the detector and said second elliptical polarization means to suppress or reflect radiation having a polarization state orthogonal to said linear polarization state.

The inventors have found that a device according to the invention, wherein the device for imaging hairs is arranged to supply elliptically polarized optical radiation, provides a much more consistent detection of hairs. The contrast between a hair and surrounding tissue is good. Moreover, and more importantly, the contrast is substantially independent of the orientation of the hair with respect to the incoming polarization. This makes detection much more reliable and any possible subsequent operation on the detected hairs more efficient.

The inventors found that the interaction of linearly polarized light with a hair depends on the orientation of the hair with respect to the polarization direction. As a result thereof, hair orientation significantly affects hair detection efficiency, which in its turn significantly decreases e.g. shaving quality, in particular during individual shaving of hairs, such as by optical means. In practice the contrast between hair and surrounding tissue may vary from hardly present to very good, which is not useful. It is assumed that the imaging becomes independent of light polarization orientation due to the fact that elliptically polarized light consists of two orthogonal, linearly polarized waves shifted in phase by 90°, such that the electric vector spirals in a helical fashion with an elliptical cross-section.

In practice, it is important to remove from the detection signal that light that is partially reflected at the interface between the skin and a medium, such as air, water, an index-matching medium, or the like, since the reflected signal is typically much stronger than the detection signal, which prevents an efficient detection.

The light that is reflected at the medium-skin interface is reflected from a dielectric surface with a reflective index that is higher than the reflective index of a light propagation medium, which is, for example, air with n ≈ 1 or water with n ≈ 1.33. This introduces a 180° phase shift of one of the components of elliptically or circularly polarized light, typically the s-component. As a consequence, the elliptically polarized light that is reflected at the medium-skin interface changes the direction of polarization, from right-hand to left-hand or vice versa. Since this reflected light does not provide information useful for imaging hairs, it is advantageous to suppress or absorb this light. This may be done with the use of e.g. a second polarization filter with a suitable polarizing action and orientation, in particular comprising an optical retardation plate.

Contrarily, part of the light that has interacted with, e.g. been scattered by a hair of interest does not change its polarization from left- to right-hand other vice versa. Since this is different from the polarization state of light reflected at the medium-skin interface, this distinction is used to improve the ratio between object-interacted and skin-reflected light, e.g. by means of the ratio increaser means.

In the context of the present invention, the source may emit radiation in the indicated wavelength range, or may alternatively emit in wavelength ranges outside said range. For efficiency reasons, however, it is advantageous if the source emits substantially only in the indicated wavelength range. A substantially monochromatic source is capable of further improving efficiency and/or accuracy.

For the present application, optical radiation with the indicated wavelength will sometimes be indicated as "light" and sometimes as "optical radiation" within the indicated wavelength ranges, this is intended to mean the same thing.

The detector may be arranged to detect area wise, i.e. to image a two-dimensional area substantially simultaneously. It is, however, also possible that the detector is arranged to detect consecutively, for example in a scanning mode, such as by scanning a light beam with respect to the object or vice versa. This will be elucidated below, where also further explanations may be found concerning embodiments that are defined in the dependent claims.

In the embodiment, wherein the ratio increaser means comprises a second elliptical polarization means, the optical radiation returning from the body part to be imaged are sent through the second elliptical polarization means. The type and the parameters, e.g. orientation, of the second elliptical polarization means, also called elliptical polarizer herein, may be selected appropriately in order to increase the contrast between desired and undesired optical radiation.

In the embodiment, wherein the ratio increaser means comprises the elliptical polarization means, use is made of the polarizing properties of the elliptical polarization means itself, as follows. As discussed above, the elliptically-polarized light reflected by the skin has a polarization state opposite to that of the incident radiation, e.g. left-hand versus right hand or vice versa. In particular, if the elliptical polarization means produces circular polarized light from linear polarized light, the polarization state of the radiation reflected at the medium-skin interface will be converted by the elliptical polarization means back into the linear state, but it will now be orthogonal to the original radiation. At the same time, the light that has interacted with hair will preserve its direction of rotation and will be converted by the polarization means back into linearly polarized light, which will have the same linear polarization as initially. As a result, the linear polarization states of the radiation reflected by the skin and interacted with a hair will be orthogonal. Therefore, the reflected light can now be efficiently suppressed, in particular by the linear polarizer. In particular, a polarizing beam splitter cube may be used as the linear polarizer, which will reflect and transmit different polarization components differently. The light reflected by the skin and affected by a hair can thus be spatially separated. In particular, the wavelength is between 800 and 1700 nm, preferably excluding the ranges 970 ± 20 nm, 1160 ± 20 nm, and 1440 ± 20 nm. It was found that the indicated wavelength range and preferably outside the three indicated sub-ranges, offers a good penetration into the skin, while still achieving a good contrast with hairs to be imaged.

In an embodiment, the elliptical polarization means has an axial ratio of between 2:1 and 1:1, inclusive, and preferably of substantially 1:1. This means that the polarization means is able to convert light with an arbitrary polarization state, e.g. linear or unpolarized, into elliptically polarized light with the indicated axial ratio, the amplitude of the light wave in a first direction then being at the most twice the amplitude of the light wave in the perpendicular direction, and preferably substantially the same. This last particular case relates to circularly polarized light. The more the axial ratio approaches 1:1, the more constant the contrast in the detected image will be.

In particular, the elliptical polarization means comprises a linear polarizer and an optical retardation plate, such as a quarter-wave plate. This offers freedom of design for the device according to the invention. For example, these parts may be positioned at a mutual distance, with one or more other elements positioned between them, as will be elucidated below. An optical retardation plate has its usual meaning herein of a plate which is transparent to the used optical radiation and which has the property that the speed of propagation for a polarization direction in a first orientation (the "fast" axis) is higher than in the direction perpendicular thereto (the "slow" axis). This causes a phase difference between the two component parts of a light wave along those two directions. If the appropriate angle with respect to the direction of polarization of the linearly polarized light and the thickness of the retardation plate, which determines the phase difference, are suitably selected, the net result will be that the light becomes elliptically polarized. It is also possible to make circularly polarized light in a manner known to those skilled in the art.

In a special embodiment, the device comprises an optical imaging system that comprises an object optical element positioned at an object side of the optical path between the skin surface and the detector and a detector optical element positioned at a detector side of the optical path. This embodiment is well suited to suppress much unwanted radiation, e.g. in order to obtain information from a certain depth within the skin without having other skin layers contribute to the signal, by imaging only a well-defined portion of the body part. The object optical element may comprise, for example, a lens, and the detector optical element may comprise a pinhole or lens. Such elements are suitable to limit the field of view of the detector, for example in order to block light coming from other parts at different depths.

In particular, the device is arranged as a confocal imaging device. In the case of a confocal imaging device, a focal point of a focusing lens is imaged on a confocal pinhole and transmitted through it. Only the signal generated in a focal point, i.e. at the position of the object of interest, is detected in this manner. Light originating outside of a focal plane, i.e. carrying no information about the object of interest, is focused before or after the pinhole and is rejected. This provides spatial resolution in the axial direction, i.e. in depth.

Advantageously, the device according to the invention further comprises a control unit for receiving a signal from the detector and for processing said signal into an image of said body part. Preferably, the control unit comprises means, such as an instruction code or software, for processing such an image, or at least detection signals, in order to recognize a hair in the body part that is imaged. In particular, the control unit is arranged to detect at least one hair in the body part that is imaged. Hair detection itself is known in the art, and details of such known hair detection means may be incorporated in the device of the present invention.

Since, according to the invention, the hair detection reliability is increased, the reliability and efficiency of the device for reducing hair growth will also be increased. In the context of the present invention, reducing hair growth may be achieved by severing the hair or by inflicting sufficient damage on the hair for it to be shed, or by stimulating dormancy of the hair-generating tissue, etc. These methods comprise epilation and shaving, for example.

The device for reducting hair growth according to the invention may advantageously comprise means for scanning a body part, advantageously a surface or subsurface area thereof, means for processing the image signal(s), and means for supplying an amount of energy to one or more detected hairs that is sufficient to reduce the growth thereof. This may be done, for example, by first imaging the body part as a whole, processing, and supplying energy. The steps may be performed substantially simultaneously. For example, a linear scan is made, a maximum brightness signal is detected, and one or more energy pulses are supplied to a location corresponding to that maximum. Note that it may happen that a hair image provides first a bright signal at a hair-skin interface, followed by a relatively low intensity signal from the cortex, followed by a very bright signal from the medulla, and symmetrically back. This gives three peaks, with the strongest in the middle. In other cases a hair may appear as a homogeneous bright object. In such a case, supplying energy could be optimized accordingly. Other rules and methods may also be used.

Any hair growth reducing means may be used in the device for reducing growth of hairs. In particular, a hair growth reducing means that affects hair subcutaneously is comprised in the device for reducing growth of hairs according to the invention. In particular, a hair growth reducing means comprises a means for providing sufficient optical energy to damage or cut the hair.

In a special embodiment, the source for imaging and the light source constitute one and the same source comprising a laser, the device further comprising a laser power control means that is arranged to switch an emitted laser power of said laser between two different non-zero values. A first value is e.g. a low value for imaging, and a second value is e.g. a high value for cutting hairs. A laser is a very suitable source of optical radiation since it is able to provide a high power density in a very small focal area, which is useful because there are high losses due to absorption and suppression of unwanted light. In various circumstances, focusing onto a small focal spot, roughly at most of the order of the diameter of the object to be imaged, is advantageous. In the case of hairs, a diameter of a few tens of micrometers is useful, although other diameters are not excluded. Such a diameter is easily achieved with a laser and a focusing lens. Other sources to be used in hair imaging are not excluded, however, for example LEDs may also be useful, because they too can provide more or less monochromatic light in useful wavelength areas, and with relatively high power. However, focusing below about 0.1 mm is not efficiently achieved. This may make the use of, for example, the pinhole advantageous.

### BRIEF DESCRIPTION OF THE DRAWINGS

The invention will now be elucidated with reference to the drawings, which show a number of non-limiting embodiments, and in which:
Fig. 1 shows a highly diagrammatic embodiment of a device for imaging hairs suitable for use in a device for reducing growth of hairs according to the invention; and
Fig. 2 diagrammatically shows an embodiment of a device for reducing growth of hairs according to the invention.

### DETAILED DESCRIPTION OF EXAMPLES

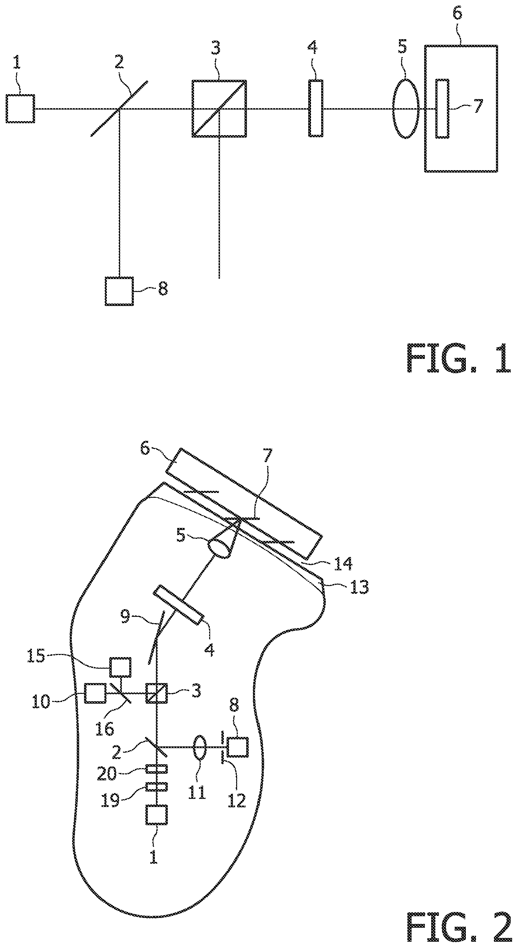

Figure 1 shows a highly diagrammatic embodiment of a device for imaging hairs suitable for use in a device for reducting growth of hairs according to invention. Herein, 1 is a light source for imaging, 2 denotes a beam splitter, 3 denotes a polarizing beam splitter, 4 denotes a quarter-wave plate, 5 denotes an imaging lens, 6 denotes tissue, 7 denotes a hair, and 8 denotes a detector.

The light source 1, such as a LED, but preferably a laser, emitting near infrared radiation of a wavelength of e.g. 834 or 1310 nm, emits with a linear polarization, e.g. in a p-state. If necessary, a linear polarizer may be added. The beam passes the beam splitter 2 and the polarizing beam splitter 3. Next, the beam passes a quarter-wave plate 4 and becomes circularly polarized, at least if the electric field factor of the beam is oriented at an angle of 45° to the fast axis of the plate 4. Next, the beam passes the imaging lens 5 and enters the tissue 6, such as skin. On entering, a major portion of the radiation will be reflected. The portion that enters the tissue 6 will be partly returned from hair 7 through scattering, reflection, or by some other mechanism.

The returning radiation will again go through imaging lens 5 and through quarter-wave plate 4. Then, there will be a difference between light reflected from the medium-skin interface and light returning from the hair 7. Light reflected at the medium - skin interface will change the direction of circular polarization, such as from left-hand to right-hand. When this light propagates through the plate 4, the resulting polarization state will be orthogonal to that of the incoming state, in this case s instead of p. This light will not pass through the polarizing beam splitter 3 but will be reflected and thus removed from the beam that travels to the detector 8.

Contrarily, at least part of the light scattered by the hair will preserve its direction of rotation and will thus be converted by the first polarization means back into the linear polarization, which will be the same as the initial polarization, in this case the p-state. The polarizing beam splitter 3 will transmit this light, which then travels to the beam splitter 2, which will reflect part of the light to the detector 8. Summarizingly, the light reflected at the medium-skin interface will be suppressed, whereas light returning from the hair 7 will be transmitted towards the detector 8, and their ratio will be improved, i.e. decreased. The hair detection efficiency is found to be independent of hair orientation.

Figure 2 diagrammatically shows an embodiment of a device for reducting hair growth according to the invention.Herein, as in all of the drawings, similar parts are denoted by the same reference numerals. The device furthermore comprises a mirror 9 for adjusting the light path, a second photodetector 10, a detector lens 11, and a pinhole 12 in front of the "first" detector 8. Furthermore, there is provided a contact window 13, while 14 denotes immersion fluid.

15 denotes a light source for severing hairs, 16 is a second beam splitter. 19 denotes a linear polarizer and 20 denotes a half-wave plate.

The present device, in the form of an ordinary shaver, comprises the parts already mentioned in the description of figure 1 and many more, the function of which will be explained below.

Radiation emitted by the light source 1 passes through a linear polarizer 19 to provide linearly polarized light in case the light source 1 would emit substantially non-polarized light. In practice, light sources such as superluminescent diodes (SLD) or a superconductor optical amplifier (SOH) may be used, which have a short coherent length and are capable of emitting elliptically polarized light with a very high axial ratio, e.g. of about 20:1, which almost represent linearly polarized light. The half-wave plate 20 may be used to rotate the linear polarization state to a preferred orientation.

Again, the beam will travel through the beam splitter 2 and the polarizing beam splitter 3 and will then hit the mirror 9 for adjusting the beam. This beam may be used for scanning the surface of the tissue 6, e.g. in two dimensions.

The reflected radiation will be split by the polarizing beam splitter 3 into radiation "mainly" reflected from the skin and radiation returned from a hair 7. The skin-reflected radiation will travel towards second detector 10, which may be a photodiode or other photodetector. If this detector receives a signal, this may indicate that an object, of course preferably skin, is present in front of the device. In other words, this second detector 10 may serve as a proximity detector.

The radiation returned from a hair 7 will travel via beam splitter 2 and detector lens 11 trough pinhole 12 to the detector 8. The detector lens 11 and the pinhole 12 serve to image a focal point of the imaging lens 5 onto the detector in order to increase the ratio of desired radiation containing information on the presence of hairs to radiation from neighboring parts that do not contain any useful information.

Not shown is a control unit for processing the signals from the detector 8. Such a control unit, which may also control the various light sources, mirrors, etc., may be embodied as a suitable IC or the like. This control unit may also comprise image processing software, e.g. for hair recognition.

The light source 15 for cutting hair is shown as a separate light source, such as a second laser with a relatively higher power. Alternatively, source 15 and source 1 may be one and the same source, but with a switchable power. The radiation from the light source 15 for cutting hairs is projected into the beam via an additional beam splitter 16. If necessary, a linear polarizer may be provided. If the proximity sensor 10 is not used, the light source 15 may also be coupled into the beam directly, i.e. without a second beam splitter 16.

The optical or contact window 13 and the immersion fluid 14, which are optional, may serve to improve the penetration properties of the radiation into the skin. For example, the fluid 14 may be an index matching fluid, having an index of refraction which is halfway between that of the optical window and that of the skin. Preferably, all refractive indices are substantially equal. This also lowers the reflection from the skin. The fluid may also be selected for the purpose of cooling the skin, or treating it otherwise. Furthermore, although the contact window 13 is optional, it helps in serving as a reference for determining positions of skin objects, such as the hairs 7.

The embodiment shown here is an example of a device that uses confocal detection to reject out-of-focus light and to obtain depth-resolved information.

## Claims

1. A device for reducing growth of hairs (7), comprising a hair growth reducing means that is arranged to supply an amount of optical energy to at least a portion of at least one hair (7) that is sufficient to affect the hair's integrity, and further comprising a device for imaging hairs (7) near a skin surface (6) of a body part, wherein the hair growth reducing means is operatively coupled to the device for imaging hairs,
**characterized in that** the device for imaging hairs (7) comprises:
- a source (1) arranged to emit optical radiation having a wavelength between 600 and 2000 nm and a linear polarization state;
- a detector (8) arranged to detect optical radiation returning from said hair (8);
- an elliptical polarization means (4) positioned in an optical path of the optical radiation between the source (1) and said skin surface (6); and
- a ratio increaser means (3, 4) that is arranged to increase a ratio of optical radiation that returns from said hairs (7) to optical radiation that returns from said skin surface (6);
wherein said ratio increaser means (3, 4) is positioned in an optical path of the optical radiation between said skin surface (6) and said detector (8) and comprises:
- either said elliptical polarization means (4) and a linear polarizer (3) arranged in the optical path between the detector (8) and said elliptical polarization means (4) to suppress or reflect radiation having a polarization state orthogonal to said linear polarization state; or
- a second elliptical polarization means and a linear polarizer (3) arranged in the optical path between the detector (8) and said second elliptical polarization means to suppress or reflect radiation having a polarization state orthogonal to said linear polarization state.

2. The device according to claim 1, wherein the wavelength is between 800 and 1700 nm, preferably excluding the ranges of 970 ± 20 nm, 1440 ± 20 nm, and 1160 ± 20 nm.

3. The device according to claim 1, wherein the elliptical polarization means (4) has an axial ratio of between 2:1 and 1:1, inclusive, and preferably of substantially 1:1.

4. The device according to claim 1, wherein the elliptical polarization means comprises a linear polarizer (19) and an optical retardation plate (4).

5. The device according to claim 1, comprising an optical imaging system that comprises an object optical element (5) positioned at an object side of the optical path between said skin surface (6) and said detector (8) and a detector optical element (8, 10) positioned at a detector side of said optical path.

6. The device according to claim 1, arranged as a confocal imaging device.

7. The device according to claim 1, further comprising a control unit for receiving a signal from the detector (8, 10) and for processing said signal into an image of said body part.

8. The device according to claim 1, wherein the source (1) comprises a laser (1, 15), the device further comprising a laser power control means that is arranged to switch an emitted laser power between two different non-zero values.

9. The device according to claim 1, wherein said linear polarizer is a polarizing beam splitter (3).

## Patentansprüche

1. Vorrichtung zur Reduzierung des Wuchses von Haaren (7), umfassend ein Haarwuchsreduzierungsmittel, das so angeordnet ist, dass es zumindest einem Teil von mindestens einem Haar (7) eine Menge an optischer Energie zuführt, die ausreicht, um auf die Integrität des Haares einzuwirken, und weiterhin umfassend eine Vorrichtung zur Abbildung von Haaren (7) nahe einer Hautoberfläche (6) eines Körperteils, wobei das Haarwuchsreduzierungsmittel mit der Vorrichtung zur Abbildung von Haaren betriebsbereit gekoppelt ist,
**dadurch gekennzeichnet, dass** die Vorrichtung zur Abbildung von Haaren (7) umfasst:
- eine Quelle (1), die so eingerichtet ist, dass sie optische Strahlung mit einer Wellenlänge zwischen 600 und 2000 nm und einem linearen Polarisationszustand emittiert;
- einen Detektor (8), der so eingerichtet ist, dass er von dem Haar (7) zurückkommende optische Strahlung detektiert;
- ein elliptisches Polarisationsmittel (4), das in einem optischen Weg der optischen Strahlung zwischen der Quelle (1) und der Hautoberfläche (6) positioniert ist; sowie
- ein Verhältniserhöhungsmittel (3, 4), das so eingerichtet ist, dass es ein Verhältnis optischer Strahlung, die von den Haaren (7) zurückkommt, auf optische Strahlung erhöht, die von der Hautoberfläche (6) zurückkommt;
wobei das Verhältniserhöhungsmittel (3, 4) in einem optischen Weg der optischen Strahlung zwischen der Hautoberfläche (6) und dem Detektor (8) positioniert ist und umfasst:
- beide, das elliptische Polarisationsmittel (4) und einen linearen Polarisator (3), der in dem optischen Weg zwischen dem Detektor (8) und dem elliptischen Polarisationsmittel (4) angeordnet ist, um Strahlung mit einem Polarisationszustand orthogonal zu dem linearen Polarisationszustand zu unterdrücken oder zu reflektieren; oder
- ein zweites elliptisches Polarisationsmittel und einen linearen Polarisator (3), der in dem optischen Weg zwischen dem Detektor (8) und dem zweiten elliptischen Polarisationsmittel angeordnet ist, um Strahlung mit einem Polarisationszustand orthogonal zu dem linearen Polarisationszustand zu unterdrücken oder zu reflektieren.

2. Vorrichtung nach Anspruch 1, wobei die Wellenlänge zwischen 800 und 1700 nm liegt, vorzugsweise ausschließlich der Bereiche von 970 ± 20 nm, 1440 ± 20 nm und 1160 ± 20 nm.

3. Vorrichtung nach Anspruch 1, wobei das elliptische Polarisationsmittel (4) ein axiales Verhältnis zwischen 2:1 und einschließlich 1:1, vorzugsweise von im Wesentlichen 1:1, aufweist.

4. Vorrichtung nach Anspruch 1, wobei das elliptische Polarisationsmittel einen linearen Polarisator (19) und eine optische Verzögerungsplatte (4) umfasst:

5. Vorrichtung nach Anspruch 1, umfassend ein optisches Bildgebungssystem, das ein optisches Objektelement (5), das auf einer Objektseite des optischen Weges zwischen der Hautoberfläche (6) und dem Detektor (8) positioniert ist, sowie ein optisches Detektorelement (8, 10), das auf einer Detektorseite des optischen Weges positioniert ist, umfasst.

6. Vorrichtung nach Anspruch 1, die als eine konfokale Bildgebungsvorrichtung eingerichtet ist.

7. Vorrichtung nach Anspruch 1, die weiterhin eine Steuereinheit zum Empfangen eines Signals von dem Detektor (8, 10) und zum Verarbeiten des Signals in ein Bild des Körperteils umfasst.

8. Vorrichtung nach Anspruch 1, wobei die Quelle (1) einen Laser (1, 15) umfasst, wobei die Vorrichtung weiterhin ein Laserleistungssteuermittel umfasst, das so eingerichtet ist, dass es eine emittierte Laserleistung zwischen zwei verschiedenen Nicht-Null-Werten schaltet.

9. Vorrichtung nach Anspruch 1, wobei der lineare Polarisator ein Polarisationsstrahlteiler (3) ist.

## Revendications

1. Dispositif permettant de réduire la pousse des poils (7), comprenant un moyen de réduction de pousse des poils qui est disposé pour fournir une quantité d'énergie optique à au moins une partie d'au moins un poil (7), laquelle est suffisante pour affecter l'intégrité du poil, et comprenant en outre un dispositif d'imagerie de poils (7) près d'une surface de peau (6) d'une partie de corps, dans lequel le moyen de réduction de pousse des poils est couplé fonctionnellement au dispositif d'imagerie de poils, **caractérisé en ce que** le dispositif d'imagerie de poils (7) comprend :
- une source (1) disposée pour émettre un rayonnement optique possédant une longueur d'onde entre 600 et 2000 nm et un état de polarisation linéaire ;
- un détecteur (8) disposé pour détecter un rayonnement optique revenant dudit poil (7) ;
- un moyen de polarisation elliptique (4) positionné dans une trajectoire optique du rayonnement optique entre la source (1) et ladite surface de peau (6) ; et
- un moyen d'augmentation de rapport (3, 4) qui est disposé pour augmenter un rapport de rayonnement optique qui revient desdits poils (7) au rayonnement optique qui revient de ladite surface de peau (6) ;
dans lequel ledit moyen d'augmentation de rapport (3, 4) est positionné dans une trajectoire optique du rayonnement optique entre ladite surface de peau (6) et ledit détecteur (8) et comprend :
- soit ledit moyen de polarisation elliptique (4) et un polariseur linéaire (3) disposés dans la trajectoire optique entre le détecteur (8) et ledit moyen de polarisation elliptique (4) pour supprimer ou réfléchir le rayonnement possédant un état de polarisation orthogonal audit état de polarisation linéaire ; soit
- un second moyen de polarisation elliptique et un polariseur linéaire (3) disposés dans la trajectoire optique entre le détecteur (8) et ledit second moyen de polarisation elliptique pour supprimer ou réfléchir le rayonnement possédant un état de polarisation orthogonal audit état de polarisation linéaire.

2. Dispositif selon la revendication 1, dans lequel la longueur d'onde est comprise entre 800 et 1700 nm, de préférence à l'exception des plages de 970 ± 20 nm, 1440 ± 20 nm et 1160 ± 20 nm.

3. Dispositif selon la revendication 1, dans lequel le moyen de polarisation elliptique (4) possède un rapport axial compris entre 2:1 et 1:1, inclus, et de préférence sensiblement égal à 1:1.

4. Dispositif selon la revendication 1, dans lequel le moyen de polarisation elliptique comprend un polariseur linéaire (19) et une plaque de retard optique (4).

5. Dispositif selon la revendication 1, comprenant un système d'imagerie optique qui comprend un élément optique objet (5) positionné au niveau d'un côté objet de la trajectoire optique entre ladite surface de peau (6) et ledit détecteur (8) et un élément optique détecteur (8, 10) positionné au niveau d'un côté détecteur de ladite trajectoire optique.

6. Dispositif selon la revendication 1, disposé en tant que dispositif d'imagerie confocal.

7. Dispositif selon la revendication 1, comprenant en outre une unité de commande pour recevoir un signal du détecteur (8, 10) et pour traiter ledit signal en une image de ladite partie du corps.

8. Dispositif selon la revendication 1, dans lequel la source (1) comprend un laser (1, 15), le dispositif comprenant en outre un moyen de commande de puissance laser qui est disposé pour commuter une puissance laser émise entre deux valeurs non nulles différentes.

9. Dispositif selon la revendication 1, dans lequel ledit polariseur linéaire est un diviseur de faisceau à polarisation (3).
